# EUROPEAN PATENT APPLICATION

(11) **EP 4 759 409 A1**
(43) Date of publication of application: **17.06.2026**
(21) Application number: 24851696.5
(22) Date of filing: 31.07.2024
(51) Int. Cl.: B01J 37/00, B01J 29/40

(54) **DESIGN SUPPORT DEVICE, LEARNING DEVICE, DESIGN SUPPORT METHOD, LEARNING METHOD, AND PROGRAM**

(30) Priority: 10.08.2023 JP 2023131006
(71) Applicant: Resonac Corporation, Tokyo 105-7325 (JP)
(72) Inventor: MINAMI, Takuya, Tokyo 105-7325 (JP); UEDA, Yasuyuki, Tokyo 105-7325 (JP); TAKASHI, Hiroko, Tokyo 105-7325 (JP); TEZUKA, Noriyasu, Tokyo 105-7325 (JP); SATO, Takashi, Tokyo 105-7325 (JP)
(74) Representative: Strehl & Partner mbB
(86) International application number: PCT/JP2024/027271
(87) International publication number: WO 2025/033271

(57) **Abstract**

A design support device for supporting design of a metal-supported zeolite catalyst by using a machine learning model that has learned a correspondence relationship between a zeolite parameter and a metal cation parameter, and reaction yield of a compound is provided, and a metal cation is supported by ion exchange. The design support device includes a parameter generation unit that generates a comprehensive combination of the zeolite parameter and the metal cation parameter; a prediction unit that predicts the reaction yield of the compound corresponding to the combination by using a trained machine learning model; and a candidate output unit that outputs data of a candidate metal-supported zeolite catalyst based on the predicted reaction yield of the compound corresponding to the combination.

## Description

### BACKGROUND OF THE INVENTION

The present disclosure relates to a design support device, a learning device, a design support method, a learning method, and a program.

Conventionally, the design of a metal-loaded zeolite catalyst having desired characteristics has been carried out by trial and error (for example, see Patent Document 1). Recently, a design method has been disclosed for designing a Si/Al ratio, a Cu loading amount, and a zeolite skeleton shape of a Cu-loaded zeolite catalyst using machine learning (for example, see Non-Patent Document 1).

### Related-Art Documents

### Patent Document

### Patent Document 1: Japanese Patent No. 6600082

Non-Patent Document 1: Catalysts informatics: paradigm shift towards data-driven catalyst design K. Takahashi, et. al., Chem. Commun. 59, 2222-2238 (2023).

### SUMMARY OF THE INVENTION

### Problem to be Solved by the Invention

However, Patent Document 1 and Non-Patent Document 1 do not describe efficiently designing a metal-supported zeolite catalyst having desired characteristics and using a new supported metal.

An object of the present disclosure is to provide a design support device, a learning device, a design support method, a learning method, and a program for supporting more efficiently designing a metal-supported zeolite catalyst having desired characteristics.

### Means for Solving the Problem

The present disclosure provide the following aspects:
[1] A design support device for supporting design of a metal-supported zeolite catalyst by using a machine learning model that has learned correspondence relationship between a zeolite parameter and a metal cation parameter and reaction yield of a compound, a metal cation being supported by ion exchange, includes:
   a parameter generation unit that generates a comprehensive combination of the zeolite parameter and the metal cation parameter;
   a prediction unit that predicts the reaction yield of the compound corresponding to the combination by using a trained machine learning model; and
   a candidate output unit that outputs data of a candidate metal-supported zeolite catalyst based on the predicted reaction yield of the compound corresponding to the combination.
[2] In the design support device in [1], the trained machine learning model has learned the correspondence relationship between the zeolite parameter, the metal cation parameter, and anionic species information, and the reaction yield of the compound, and
   the parameter generation unit generates the comprehensive combination of the zeolite parameter, the metal cation parameter, and the anionic species information to be input to the trained machine learning model.
[3] In the design support device in [1] or [2], the reaction yield of the compound includes at least one of propylene yield, an olefin/paraffin ratio, and useful component yield.
[4] A learning device for training a machine learning model, includes:
   a training data set acquisition unit that acquires a training data set representing correspondence relationship between a zeolite parameter and a metal cation parameter and reaction yield of a compound, a metal cation being supported by ion exchange; and
   a training unit that trains the machine learning model based on the training data set by using the zeolite parameter and the metal cation parameter as explanatory variables and the reaction yield of the compound as an objective variable.
[5] In the learning device in [4], the training data set represents the correspondence relationship between the zeolite parameter, the metal cation parameter, and anion species information, and the reaction yield of the compound, and
   the training unit trains the machine learning model based on the training data set by using the zeolite parameter, the metal cation parameter, and the anionic species information as explanatory variables and the reaction yield of the compound as the objective variable.
[6] In the learning device in [4] or [5], the reaction yield of the compound includes at least one of propylene yield, an olefin/paraffin ratio, and useful component yield.
[7] A design support method for supporting design of a metal-supported zeolite catalyst using a machine learning model in which a computer has learned correspondence relationship between a zeolite parameter and a metal cation parameter, and reaction yield of a compound, a metal cation being supported by ion exchange, includes:
   a parameter generation step of generating a comprehensive combination of the zeolite parameter and the metal cation parameter;
   a prediction step of predicting the reaction yield of the compound corresponding to the combination by using a trained machine learning model; and
   a candidate output step of outputting data of a candidate metal-supported zeolite catalyst based on the predicted reaction yield of the compound corresponding to the combination.
[8] A design support method executed by a computer for training a machine learning model, includes:
   a training data set acquisition step of acquiring a training data set representing correspondence relationship between a zeolite parameter and a metal cation parameter, and reaction yield of a compound, a metal cation being supported by ion exchange; and
   a training step of training the machine learning model based on the training data set by using the zeolite parameter and the metal cation parameter as explanatory variables, and the reaction yield of the compound as an objective variable.
[9] A program causing a computer, for supporting design of a metal-supported zeolite catalyst using a machine learning model that has learned correspondence relationship between a zeolite parameter and a metal cation parameter, and reaction yield of a compound, a metal cation being supported by ion exchange, to execute:
   a parameter generation procedure of generating a comprehensive combination of the zeolite parameter and the metal cation parameter;
   a prediction procedure of predicting the reaction yield of the compound corresponding to the combination by using a trained machine learning model; and
   a candidate output procedure of outputting data of a candidate metal-supported zeolite catalyst based on the predicted reaction yield of the compound corresponding to the combination.
[10] A program causing a computer for learning a machine learning model to execute:
   a training data set acquisition procedure of acquiring a training data set representing correspondence relationship between a zeolite parameter and a metal cation parameter, and reaction yield of a compound, a metal cation being supported by ion exchange; and
   a training procedure of training the machine learning model based on the training data set by using the zeolite parameter and the metal cation parameter as explanatory variables, and the reaction yield of the compound as an objective variable.

According to the present disclosure, it is possible to provide a design support device, a learning device, a design support method, a learning method, and a program that support more efficient design of a metal-supported zeolite catalyst having desired characteristics.

### BRIEF DESCRIPTION OF THE DRAWINGS

[FIG. 1] FIG. 1 is a diagram illustrating a configuration example of an information processing system according to the present embodiment.
[FIG. 2] FIG. 2 is a diagram illustrating a hardware configuration example of a computer according to the present embodiment.
[FIG. 3] FIG. 3 is a diagram illustrating a functional configuration example of the information processing system according to the present embodiment.
[FIG. 4] FIG. 4 is a flowchart illustrating an example of processing for learning a machine learning model in the information processing system according to the present embodiment.
[FIG. 5] FIG. 5 is a diagram illustrating a configuration example of a training data set before parameter conversion.
[FIG. 6] FIG. 6 is a diagram illustrating a configuration example of a parameter table of metal cations.
[FIG. 7] FIG. 7 is a diagram illustrating a configuration example of the training data set after parameter conversion.
[FIG. 8] FIG. 8 is a flowchart illustrating an example of a process for predicting candidates of metal-supported zeolite catalysts having desired characteristics in the information processing system according to the present embodiment.
[FIG. 9] FIG. 9 is a diagram illustrating an example of a search range for metal-supported zeolite catalysts.
[FIG. 10] FIG. 10 is a block diagram illustrating an example of a parameter table.
[FIG. 11A] FIG. 11A is a diagram for describing an example of the relationship between predicted values of a trained machine learning model and measured values of a propylene yield according to the present embodiment.
[FIG. 11B] FIG. 11B is a diagram for describing an example of the relationship between predicted values of the trained machine learning model and measured values of a useful component yield according to the present embodiment.
[FIG. 12] FIG. 12 is a diagram illustrating data of a candidate metal-supported zeolite catalyst having a high prediction result of a propylene yield or a useful component yield.
[FIG. 13A] FIG. 13A is a diagram illustrating an example of an experimental result of the propylene yield.
[FIG. 13B] FIG. 13B is a diagram illustrating an example of the experimental result of a C2-5 olefin/paraffin ratio.
[FIG. 13C] FIG. 13C is a diagram illustrating an example of the experimental result of a useful component yield.
[FIG. 14] FIG. 14 is a diagram illustrating an experimental result when Na+, Sr2+, or Rb+ is used as the metal cation.

### DESCRIPTION OF THE PREFERRED EMBODIMENTS

Next, embodiments of the present invention will be described in detail. The present invention is not limited to the following embodiments.

### [First Embodiment]

### <System Configuration>

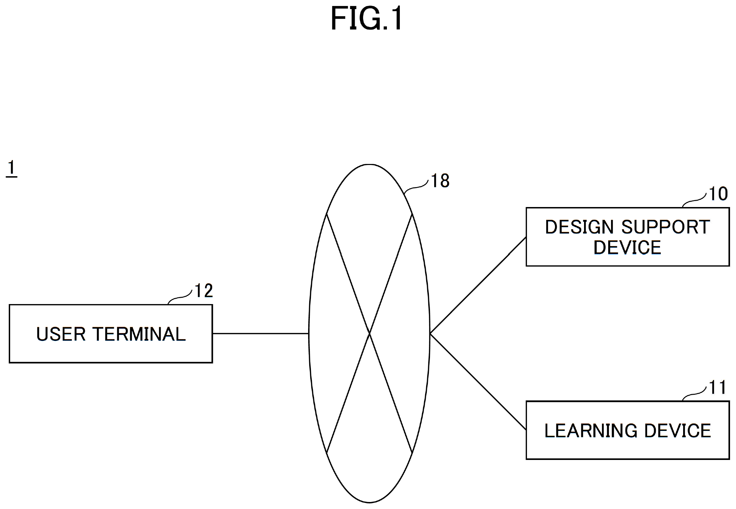

FIG. 1 is a diagram illustrating a configuration example of an information processing system according to the present embodiment. An information processing system 1 of FIG. 1 includes a design support device 10, a learning device 11, and a user terminal 12. The design support device 10, the learning device 11, and the user terminal 12 are connected to one another via a communication network 18 such as a local area network (LAN) or the Internet so as to enable data communication.

The user terminal 12 is an information processing terminal, such as a PC, a tablet terminal, or a smartphone, operated by an operator. The user terminal 12 displays a screen on a display device for accepting information input from an operator, and accepts the information input from the operator. The user terminal 12 also transmits information received from the operator to the design support device 10, and causes the design support device 10 to execute processing for supporting more efficient design of a metal-supported zeolite catalyst having desired characteristics. The user terminal 12 receives information on an execution result of the processing from the design support device 10, and displays the information on the display device for the operator to confirm. The user terminal 12 also transmits the information received from the operator to the learning device 11, and causes the learning device 11 to execute processing for learning a machine learning model as described later. The user terminal 12 may receive information on an execution result of the processing from the learning device 11, and display the information on the display device for the operator to confirm.

The design support device 10 is an information processing device such as a PC that supports more efficient design of a metal-supported zeolite catalyst having desired characteristics as described later. The design support device 10 supports more efficient design of the metal-supported zeolite catalyst having desired characteristics by executing processing for outputting candidates of the metal-supported zeolite catalyst having desired characteristics.

The design support device 10 can utilize a mathematical model such as a machine learning model (trained machine learning model) in which a correspondence relationship between zeolite parameters and metal cation parameters supported by ion exchange, and a reaction yield of a compound has been learned. As a machine learning method, for example, regression or classification can be used. The design support device 10 may use a mathematical model, such as a machine learning model that has learned the correspondence relationship between zeolite parameters, metal cation parameters supported by ion exchange, and anion species information and the reaction yield of the compound. As the machine learning method, for example, regression or classification can be used.

Specifically, as the machine learning method, for example, a supervised learning method, such as linear, generalized linear (Lasso, ridge, elastic net, logistic, or the like), partial least squares, kernel ridge, Gaussian process, k-nearest neighbors, decision tree, random forest, AdaBoost, bagging, gradient boosting, a support vector machine, or a neural network, can be used.

The design support device 10 generates a comprehensive combination of zeolite parameters and metal cation parameters supported by ion exchange, and predicts the reaction yield of the compound, corresponding to the comprehensive combination using the trained machine learning model. The design support device 10 generates a comprehensive combination of zeolite parameters, metal cation parameters supported by ion exchange, and anion species information, and predicts the reaction yield of the compound, corresponding to the comprehensive combination using the trained machine learning model.

By predicting the reaction yield of the compound corresponding to such a comprehensive combination using the trained machine learning model, the design support device 10 can output a candidate metal-supported zeolite catalyst having desired characteristics.

For example, when the reaction yield of the compound is predicted by the trained machine learning model, zeolite parameters, and metal cation parameters supported by ion exchange are input to the trained machine learning model. Further, for example, when the reaction yield of the compound is predicted by the trained machine learning model, zeolite parameters, metal cation parameters supported by ion exchange, and anion species information may be input to the trained machine learning model.

The design support device 10 receives the information input by the operator to the user terminal 12, and executes processing for supporting the design of the metal-supported zeolite catalyst. The design support device 10 transmits processing result information to the user terminal 12, and causes the user terminal 12 to display the processing result information (for example, data of one or more candidate metal-supported zeolite catalysts).

The learning device 11 causes the machine learning model to learn the correspondence relationship between zeolite parameters, metal cation parameters supported by ion exchange, and the reaction yield of the compound as described later. The learning device 11 may cause the machine learning model to learn the correspondence relationship between the zeolite parameters, the metal cation parameters supported by ion exchange, and the anionic species information and the reaction yield of the compound as described later.

The information processing system 1 of FIG. 1 can be realized by the design support device 10 with a Web server function, the learning device 11, and the user terminal 12 that executes a Web application by a Web browser function. Further, the information processing system 1 of FIG. 1 may be realized by an application installed in the user terminal 12 that performs processing in cooperation with a program that is installed in the design support device 10 and the learning device 11.

The information processing system 1 of FIG. 1 is an example, and various system configuration examples are possible depending on the application and purpose. For example, the design support device 10 and the learning device 11 may be realized by a plurality of computers, may be realized as a cloud computing service, or may be realized by a single computer. Further, the information processing system 1 of FIG. 1 may be realized by a stand-alone computer.

### <Hardware Configuration>

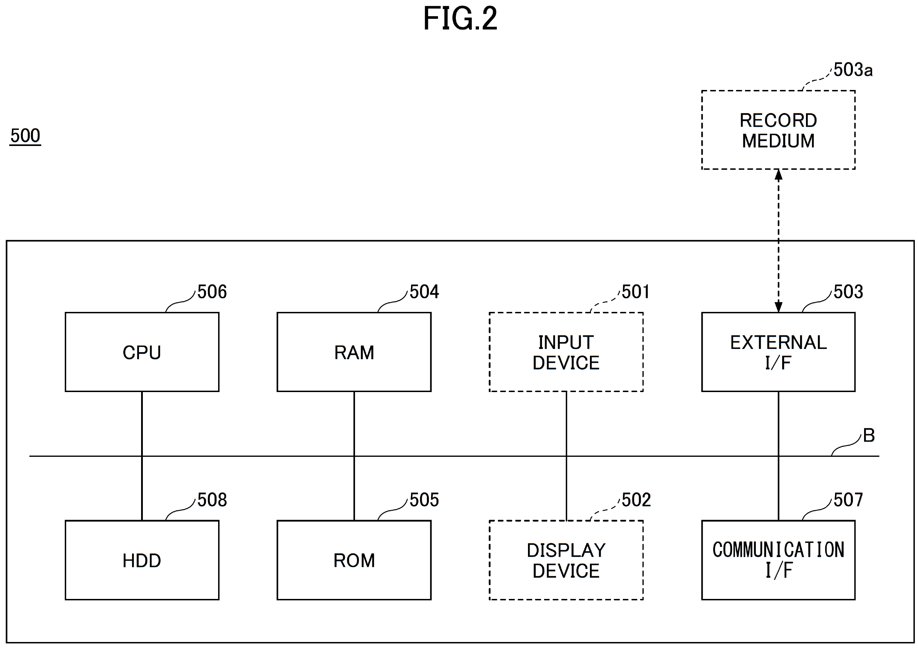

The design support device 10, the learning device 11, and the user terminal 12 of FIG. 1 may be realized by a computer 500 having a hardware configuration illustrated in FIG. 2, for example.

FIG. 2 is a diagram illustrating a hardware configuration example of the computer according to the present embodiment. The computer 500 of FIG. 2 includes an input device 501, a display device 502, an external I/F 503, a RAM 504, a ROM 505, a CPU 506, a communication I/F 507, an HDD 508, and the like, which are mutually connected by a bus B. The input device 501 and the display device 502 may be connected and used.

The input device 501 includes a touch panel, operation keys and buttons, a keyboard, a mouse, and the like which are used by a user to input various signals. The display device 502 includes a display such as of liquid crystal or organic EL for displaying a screen, and includes a speaker or the like for outputting sound data such as voice and sound. The communication I/F 507 is an interface for the computer 500 to perform data communication.

The HDD 508 is an example of a nonvolatile storage device for storing programs and data. The stored programs and data include an OS, which is basic software for controlling the entire computer 500, and include applications or the like for providing various functions on the OS. Instead of the HDD 508, the computer 500 may use a drive device (such as, for example, a solid state drive: SSD) using a flash memory as a storage medium.

The external I/F 503 is an interface with an external device. The external device includes a recording medium 503a or the like. Thus, the computer 500 can read and/or write to the recording medium 503a via the external I/F 503. The recording medium 503a includes a flexible disk, a CD, a DVD, an SD memory card, a USB memory, or the like.

The ROM 505 is an example of a nonvolatile semiconductor memory (storage device) that can hold programs and data even when the power is turned off. The ROM 505 stores programs and data such as BIOS, OS settings, and network settings that are executed when the computer 500 is started. The RAM 504 is an example of a volatile semiconductor memory (storage device) that temporarily holds programs and data.

The CPU 506 is an arithmetic device that reads programs and data from storage devices such as the ROM 505 and the HDD 508 into the RAM 504 and executes processing to realize control and functions of the entire computer 500. By executing programs, the computer 500 according to the present embodiment can realize various functions of the design support device 10, the learning device 11, and the user terminal 12.

### <Functional Configuration>

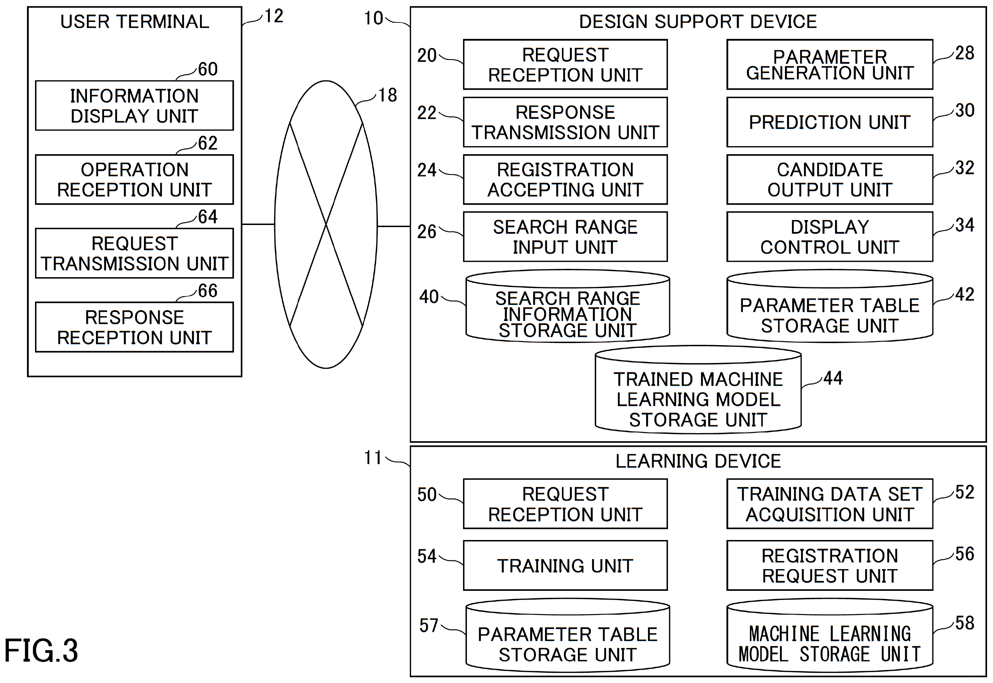

A functional configuration of the information processing system 1 according to the present embodiment will be described. FIG. 3 is a diagram illustrating a functional configuration example of the information processing system according to the present embodiment. In the configuration diagram of FIG. 3, parts unnecessary for the description of the present embodiment are appropriately omitted.

The design support device 10 includes a request reception unit 20, a response transmission unit 22, a registration accepting unit 24, a search range input unit 26, a parameter generation unit 28, a prediction unit 30, a candidate output unit 32, a display control unit 34, a search range information storage unit 40, a parameter table storage unit 42, and a trained machine learning model storage unit 44.

The learning device 11 includes a request reception unit 50, a training data set acquisition unit 52, a training unit 54, a registration request unit 56, a parameter table storage unit 57, and a machine learning model storage unit 58.

The user terminal 12 includes an information display unit 60, an operation reception unit 62, a request transmission unit 64, and a response reception unit 66.

The information display unit 60 displays, on the display device 502, a screen for accepting information input from the operator and a screen for displaying information on the execution result of processing from the design support device 10 or the learning device 11. The operation reception unit 62 accepts an operation such as information input through the operator. The request transmission unit 64 transmits a processing request corresponding to the information input from the operator, to the design support device 10 or the learning device 11. The response reception unit 66 receives a response to the processing request transmitted by the request transmission unit 64 from the design support device 10 or the learning device 11.

The request reception unit 20 receives a processing request from the user terminal 12. The response transmission unit 22 responds with the execution result of the processing corresponding to the processing request. The registration accepting unit 24 accepts registration of search range information described below from, for example, an operator operating the user terminal 12, and stores the registration in the search range information storage unit 40. The search range information storage unit 40 stores search range information described below. The registration accepting unit 24 also accepts registration of a parameter table described below from, for example, an operator operating the user terminal 12, and stores the registration in the parameter table storage unit 42. The parameter table storage unit 42 stores a parameter table described below. The registration accepting unit 24 also accepts registration of a trained machine learning model from the learning device 11, and stores the registration in the trained machine learning model storage unit 44. The trained machine learning model storage unit 44 stores the trained machine learning model. The search range input unit 26 accepts input of a comprehensive combination range generated by the parameter generation unit 28 from an operator.

The parameter generation unit 28 generates a comprehensive combination of zeolite parameters and metal cation parameters supported by ion exchange. The design support device 10 may generate a comprehensive combination of zeolite parameters, metal cations parameters supported by ion exchange, and anionic species information.

The prediction unit 30 predicts the reaction yield of a compound corresponding to the comprehensive combination generated by the parameter generation unit 28 by using a trained machine learning model.

The candidate output unit 32 outputs data of a candidate metal-supported zeolite catalyst based on the reaction yield of the compound corresponding to the comprehensive combination predicted by the prediction unit 30. The display control unit 34 performs a control such that data of the candidate metal-supported zeolite catalyst output by the candidate output unit 32 is displayed on the user terminal 12.

The request reception unit 50 receives a processing request from the user terminal 12. The training data set acquisition unit 52 acquires a training data set representing the correspondence relationship between zeolite parameters and metal cation parameters supported by ion exchange, and the reaction yield of the compound. The training data set acquisition unit 52 may acquire a training data set representing the correspondence relationship between (zeolite parameters, metal cation parameters supported by ion exchange, and anionic species information), and the reaction yield of the compound. The training data set acquisition unit 52 may acquire a parameter table described later and store the table in a parameter table storage unit 57. The parameter table storage unit 57 stores the parameter table described later.

The training unit 54 causes the machine learning model to learn the correspondence relationship between zeolite parameters and metal cation parameters supported by ion exchange, and the reaction yield of the compound, based on the training data set acquired by the training data set acquisition unit 52. The training unit 54 may cause the machine learning model to learn the correspondence relationship between (zeolite parameters, metal cation parameters supported by ion exchange, and anionic species information), and the reaction yield of the compound, based on the training data set acquired by the training data set acquisition unit 52.

The registration request unit 56 requests the design support device 10 to register the machine learning model that is trained by the training unit 54, and causes the trained machine learning model to be stored in the trained machine learning model storage unit 44. The machine learning model storage unit 58 stores the machine learning model trained by the training unit 54.

The configuration of FIG. 3 is an example. The configuration of the information processing system 1 according to the present embodiment can be realized by various configurations. For example, the search range information storage unit 40, the parameter table storage unit 42, the trained machine learning model storage unit 44, the parameter table storage unit 57, and the machine learning model storage unit 58 may include a storage device, a computer, or cloud storage capable of communicating data with the design support device 10 or the learning device 11.

### <Process>

### <<Learning Process>>

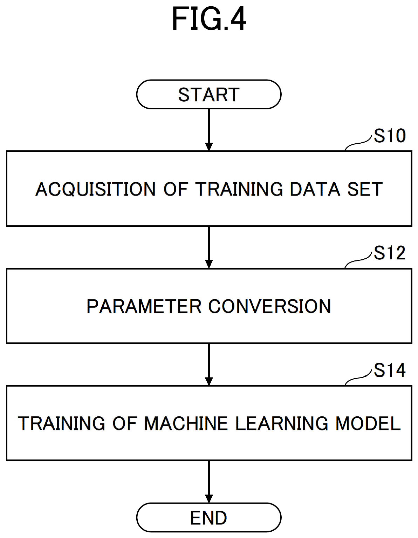

The information processing system 1 according to the present embodiment performs the learning process for the machine learning model by, for example, the procedure illustrated in FIG. 4. FIG. 4 is a flowchart illustrating an example of the process for learning the machine learning model in the information processing system according to the present embodiment. For example, the flowchart of FIG. 4 illustrates the process started upon receiving, from the user terminal 12, a request for the learning process for the machine learning model.

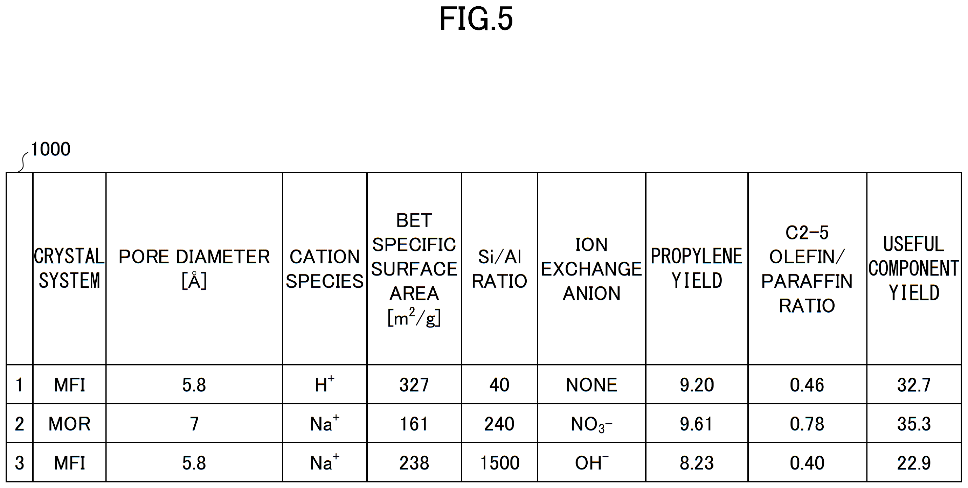

In step S10, the training data set acquisition unit 52 of the learning device 11 acquires a training data set to be used for training the machine learning model. The training data set is a training data set representing the correspondence relationship between zeolite parameters, metal cation parameters supported by ion exchange, and a reaction yield of a compound, or a training data set representing the correspondence relationship between (zeolite parameters, metal cation parameters supported by ion exchange, and anion species information), and a reaction yield of a compound. Here, for example, an example of a training data set 1000 illustrated in FIG. 5 will be described.

FIG. 5 is a diagram illustrating a configuration example of the training data set before parameter conversion. The training data set 1000 illustrated in FIG. 5 has items that include a crystal system, a pore size, a cation species, a BET specific surface area, a Si/Al ratio, ion exchange anion, a propylene yield, a C2-5 olefin/paraffin ratio, and a useful component yield.

The crystal system indicates a skeleton structure of a zeolite by a skeleton code. The pore size, the BET specific surface area, and the Si/Al ratio are examples of parameters of the zeolite skeleton. The cation species indicates a cation element. The ion exchange anion represents anion species information. The propylene yield, the C2-5 olefin/paraffin ratio, and the useful component yield indicate the reaction yield of the compound.

In step S12, the training data set acquisition unit 52 performs parameter conversion for items in the training data set acquired in step S10 that require parameter conversion. If there are no items that require parameter conversion, the processing of step S12 may be skipped.

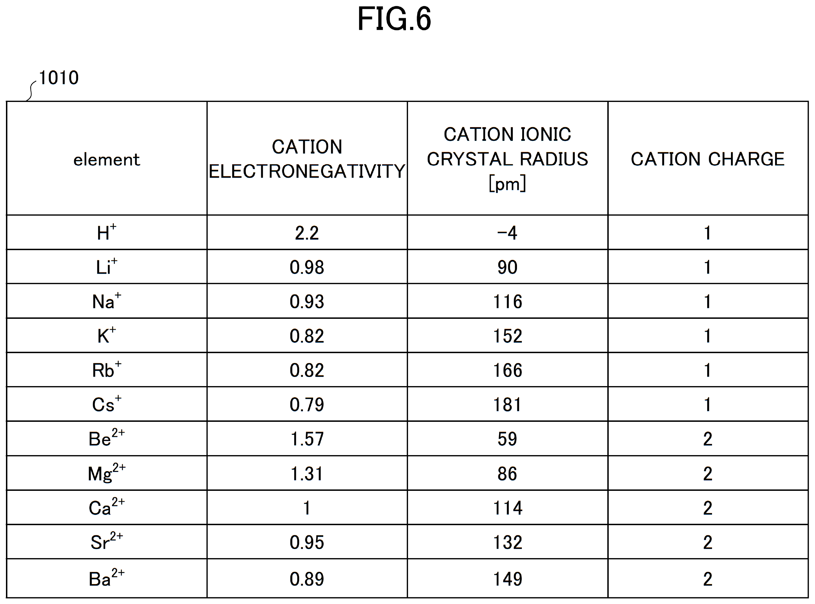

For example, in the case of the training data set 1000 of FIG. 5, the training data set acquisition unit 52 converts the data for the item "cation species" of FIG. 5 into cation electronegativity, a cation ion crystal radius, and a cation charge, which are metal cations parameters, using a parameter table 1010 of FIG. 6. FIG. 6 is a diagram illustrating a configuration example of the parameter table for metal cations.

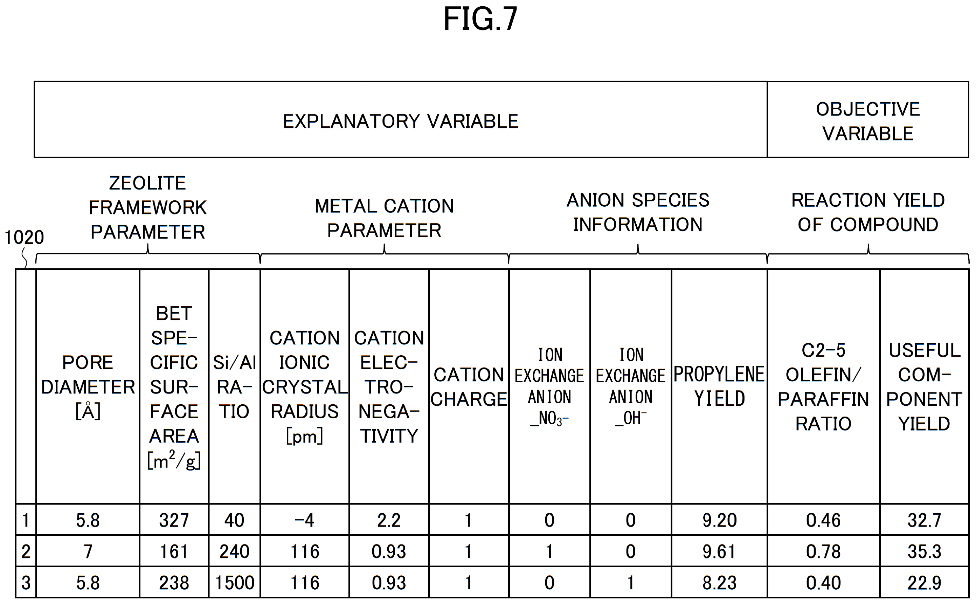

By the processing of step S12, the training data set before parameter conversion of FIG. 5 is converted into a training data set 1020 of FIG. 7. FIG. 7 is a diagram illustrating a configuration example of the training data set after parameter conversion. The training data set 1020 illustrated in FIG. 7 shows the correspondence relationship between (zeolite parameters, metal cation parameters supported by ion exchange, and anion species information), and a reaction yield of a compound. In FIG. 7, the item "anion species information" is converted into a value "0" indicating that an anion is not used or a value "1" indicating that the anion is used, as a dummy variable.

In the training data set 1020, the items "pore diameter," "BET specific surface area," and "Si/Al ratio" indicate parameters of the zeolite skeleton (examples of zeolite parameters). In the training data set 1020, the items "cation ion crystal radius," "cation electronegativity," and "cation charge" indicate metal cation parameters. In the training data set 1020, the items "ion exchange anion_NO3-" and "ion exchange anion_OH-" indicate anion species information. In the training data set 1020, the items "propylene yield," "C2-5 olefin/paraffin ratio," and "useful component yield" indicate the reaction yield of the compound.

In step S14, the training unit 54 causes the machine learning model to learn the correspondence relationship between (zeolite parameters, metal cation parameters supported by ion exchange, and anion species information), and the reaction yield of the compound, based on the training data set after parameter conversion illustrated in FIG. 7, for example.

Specifically, the training unit 54 causes the machine learning model to learn by using, for example, the zeolite parameters of the training data set after parameter conversion illustrated in FIG. 7, the metal cation parameters supported by ion exchange, and the anionic species information, as explanatory variables, and the reaction yields of the compound as objective variables.

The machine learning model that has been trained by the learning device 11 is used for prediction processing in the design support device 10.

### <<Prediction Processing>>

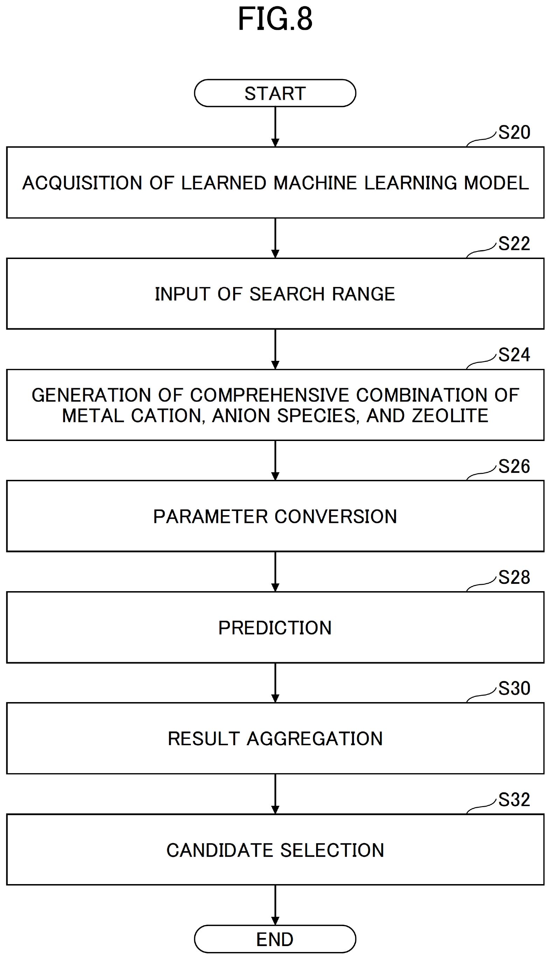

The information processing system 1 according to the present embodiment predicts a candidate of a metal-supported zeolite catalyst having desired characteristics by, for example, the procedure illustrated in FIG. 8. FIG. 8 is a flowchart illustrating an example of processing for predicting the candidate of the metal-supported zeolite catalyst having desired characteristics, in the information processing system according to this embodiment. For example, the flowchart of FIG. 8 shows processing started upon receiving a request for prediction processing from the user terminal 12.

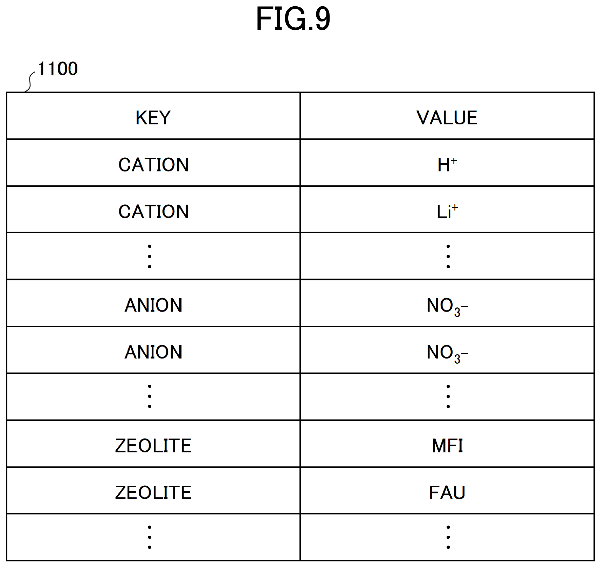

In step S20, the design support device 10 acquires the machine learning model that has been trained by the learning device 11. In step S22, the search range input unit 26 receives an input of the search range of the metal-supported zeolite catalyst. For example, the search range input unit 26 receives an input of the search range 1100 of the metal-supported zeolite catalyst illustrated in FIG. 9 from search range information stored in the search range information storage unit 40.

FIG. 9 is a diagram illustrating an example of a search range of metal-supported zeolite catalysts. The search range illustrated in FIG. 9 is an example, and a plurality of values such as the BET specific surface area or the Si/Al ratio may be specified. For example, when the BET specific surface area is included in the search range of metal-supported zeolite catalysts, 5, 100, 200, 300, 400, 500, 600, and 700 may be specified.

In step S24, the parameter generation unit 28 generates a comprehensive combination of metal cations, anions, and zeolites in the search range of the metal-supported zeolite catalyst received as input in step S22.

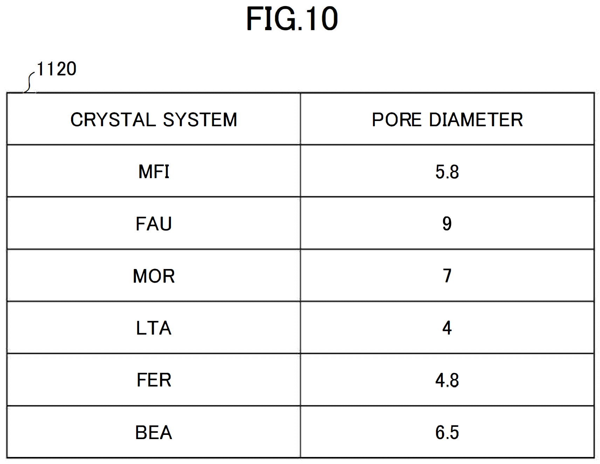

In step S26, the parameter generation unit 28 performs parameter conversion for items that need to be converted into parameters for each of the comprehensive combinations of metal cations, anions, and zeolites generated in step S24. For example, the parameter generation unit 28 converts data for the item "cation species" into cation electronegativity, a cation ion crystal radius, and a cation charge, which are metal cation parameters using the parameter table 1010 illustrated in FIG. 6. The parameter generation unit 28 also converts data for the item "crystal system" into a pore diameter, which is a parameter of the zeolite skeleton, by using the parameter table 1120 illustrated in FIG. 10. Since the BET specific surface area and Si/Al ratio are not uniquely determined for the zeolite skeleton, it is necessary to separately input numerical values.

In step S28, the prediction unit 30 inputs comprehensive combinations of metal cations, anions, and zeolites generated by the parameter generation unit 28 to the trained machine learning model to predict the reaction yield of the compound. For example, when the reaction yield of the compound indicates propylene yield, a C2-5 olefin/paraffin ratio, and useful component yield, the comprehensive combinations of metal cations, anions, and zeolites are input to the trained machine learning model for propylene yield, the trained machine learning model for the C2-5 olefin/paraffin ratio, and the trained machine learning model for the useful component yield.

In step S30, the candidate output unit 32 totalizes the reaction yield of the compound corresponding to the comprehensive combinations predicted by the prediction unit 30. For example, the candidate output unit 32 sorts the reaction yield of the compound corresponding to the comprehensive combinations predicted by the prediction unit 30 in descending order of, for example, the propylene yield or the useful component yield.

In step S32, the candidate output unit 32 outputs data of the candidate metal-supported zeolite catalyst based on the reaction yield of the compound corresponding to the comprehensive combinations predicted by the prediction unit 30. For example, the candidate output unit 32 can select and output data of the candidate metal-supported zeolite catalyst from the comprehensive combinations predicted by the prediction unit 30 having high propylene yield or high useful component yield.

### <<Example>>

When a low-molecular compound (methyl, ethyl, propyl, ethylene, propylene, or the like) is produced by decomposing a plastic, a metal-supported zeolite is used as a catalyst. By using a metal-supported zeolite as the catalyst, it is desired to increase the yield (propylene yield, useful component yield, or the like) of a desired component.

The useful component yield is the sum of the yield of olefins up to C5 and useful aromatics (benzene, toluene, ethylbenzene, styrene, xylene (o-xylene, m-xylene, and p-xylene may be added together)).

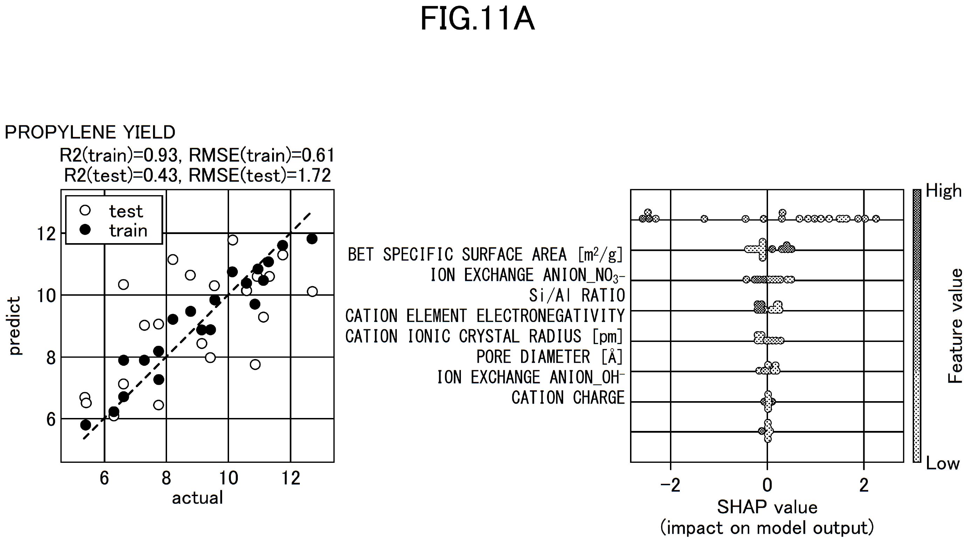

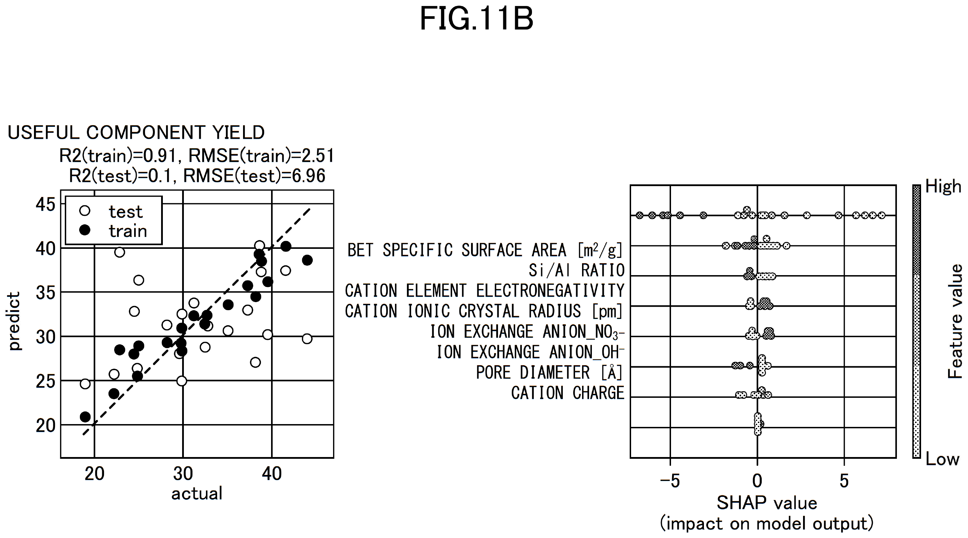

FIGS. 11A and 11B are diagrams illustrating examples of the relationships between predicted values and measured values of the trained machine learning models for propylene yield and the useful component yield according to the present embodiment. FIG. 11A illustrates the relationship between the predicted values and the measured values of the trained machine learning model for the propylene yield and a SHAP (Shapley Value) value for items of the explanatory variables. FIG. 11B illustrates the relationship between the predicted values of the trained machine learning model and the measured values of the useful component yield and illustrates the SHAP value for items of the explanatory variables. The SHAP value indicates the degree of contribution of each item of the explanatory variables to the predicted values of the trained machine learning model.

When it is desired to search for a metal cation that increases the propylene yield and the useful component yield, it is understood from the SHAP value that the propylene yield can be improved by using metal cations that have lower electronegativity and larger ionic crystal radii. In addition, when it is desired to search for a metal cation that increases the propylene yield and the useful component yield, it is understood that higher-period cations show better performance.

In the information processing system 1 according to the present embodiment, after the reaction yield of the compound is predicted for all comprehensive combinations of the search range, the combinations are sorted in descending order of propylene yield, and the "Rb+ system" is selected as the metal cation.

Further, in the information processing system 1 according to the present embodiment, the reaction yield of the compound is predicted for all exhaustive combinations of the search range, and then the combinations are sorted in descending order of useful component yield, and the "Sr2+ system" is selected as the metal cation.

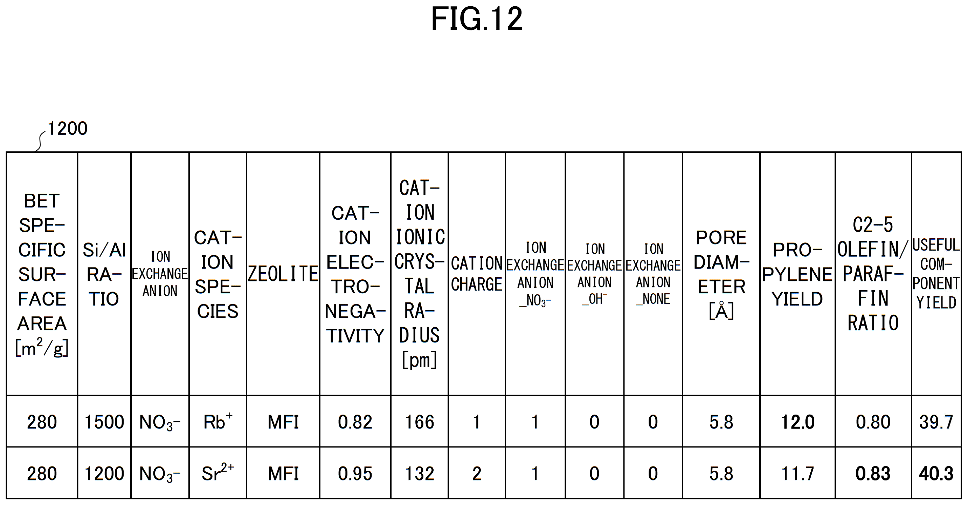

FIG. 12 is a diagram illustrating data of candidate metal-supported zeolite catalysts whose predicted propylene yields or useful component yields are high. Experiments have been conducted on data 1200 of the candidate metal-supported zeolite catalysts whose predicted propylene yields or useful component yields are high, and measured values are obtained as experimental results.

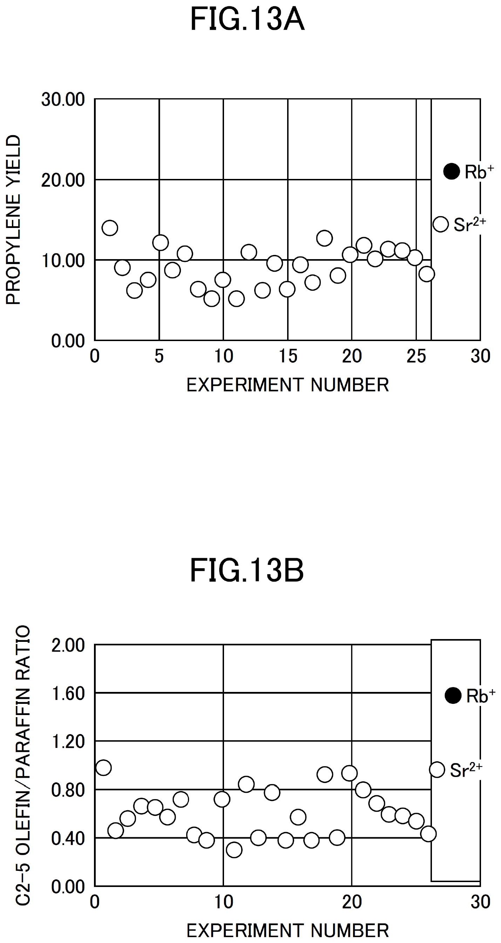

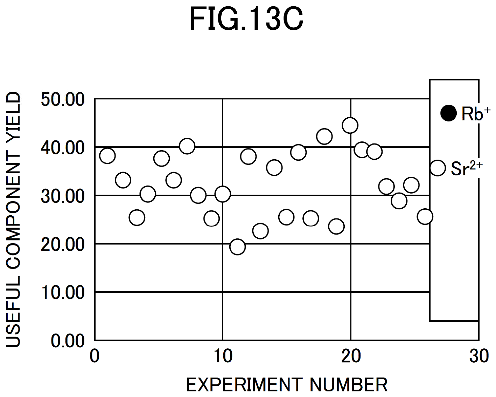

FIGS. 13A to 13C are diagrams illustrating examples of experimental results. FIG. 13A shows experimental results of propylene yields. FIG. 13B shows experimental results of C2-5 olefin/paraffin ratios. FIG. 13C shows experimental results of useful component yields.

As illustrated in FIGS. 13A to 13C, the metal-supported zeolite catalyst using the "Rb+ system" as the metal cation shows improved propylene yield, C2-5 olefin/paraffin ratio, and useful component yield. In particular, the improvement in propylene yield and C2-5 olefin/paraffin ratio are remarkable.

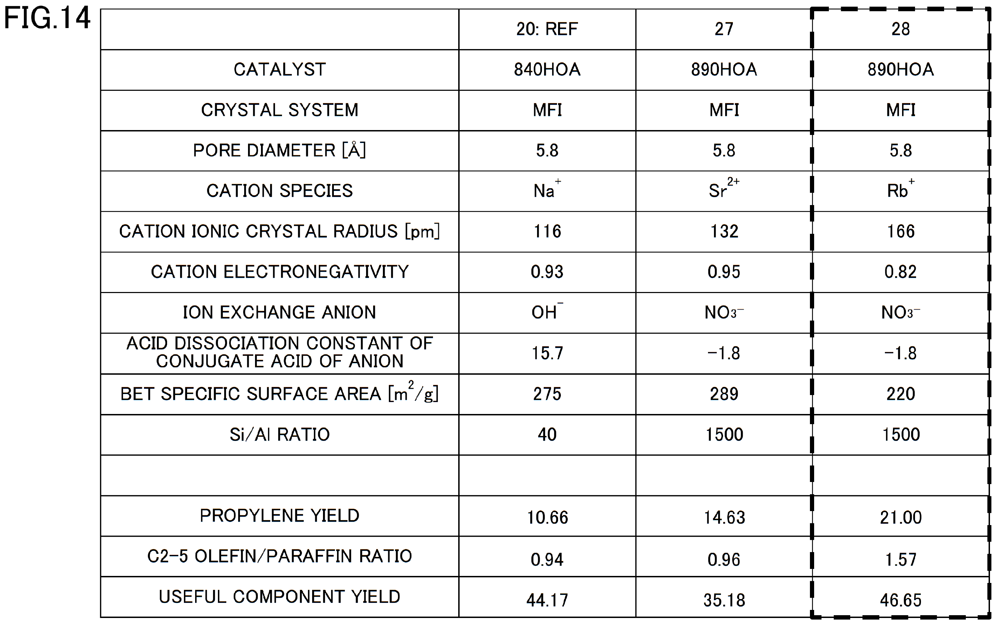

FIG. 14 is a diagram illustrating experimental results when Na+, Sr2+, or Rb+ is used as the metal cation. As illustrated in FIG. 14, the experimental results when Rb+ is used as the metal cation show higher performance than the experimental results when Na+ or Sr2+ is used as the metal cation.

In the information processing system 1 according to the present embodiment, comprehensive combinations of zeolite parameters and metal cation parameters can be screened using a trained machine learning model that has learned the correspondence relationships between zeolite parameters and metal cation parameters supported by ion exchange, and the reaction yield of the compound, and data of metal-supported zeolite catalysts having desired characteristics can be output. In the information processing system 1 according to the present embodiment, comprehensive combinations of zeolite parameters, metal cation parameters, and anion species can be screened using a trained machine learning model that has learned the correspondence relationships between (zeolite parameters, metal cation parameters supported by ion exchange, and anion species information), and the reaction yield of the compound, and data of metal-supported zeolite catalysts having desired characteristics can be output.

Therefore, in the information processing system 1 according to the present embodiment, more efficient design of metal-supported zeolite catalysts having desired characteristics can be supported.

### [Other Embodiment]

The metal-supported zeolite catalyst that is designed by the design support device 10 according to the present embodiment may generate a metal-supported zeolite catalyst having desired characteristics by supplying data of the metal-supported zeolite catalyst to a manufacturing apparatus that generates the metal-supported zeolite catalyst by blending a plurality of raw materials, for example.

Although the present embodiment has been described above, it will be understood that various changes in form and details can be made without departing from the intent and scope of the claims. Although the present invention has been described based on the embodiments, the present invention is not limited to the above embodiments and various modifications can be made within the scope of the claims. This application claims priority to basic application No. 2023-131006, filed on August 10, 2023 in the Japan Patent Office, the entire contents of which are incorporated herein by reference.

### Reference Signs List

- 1: information processing system
- 10: design support device
- 11: learning device
- 12: user terminal
- 18: communication network
- 28: parameter generation unit
- 30: prediction unit
- 32: candidate output unit
- 52: training data set acquisition unit
- 54: training unit

## Claims

1. A design support device for supporting design of a metal-supported zeolite catalyst by using a machine learning model that has learned correspondence relationship between a zeolite parameter and a metal cation parameter and reaction yield of a compound, a metal cation being supported by ion exchange, comprising:
a parameter generation unit that generates a comprehensive combination of the zeolite parameter and the metal cation parameter;
a prediction unit that predicts the reaction yield of the compound corresponding to the combination by using a trained machine learning model; and
a candidate output unit that outputs data of a candidate metal-supported zeolite catalyst based on the predicted reaction yield of the compound corresponding to the combination.

2. The design support device according to claim 1, wherein the trained machine learning model has learned the correspondence relationship between the zeolite parameter, the metal cation parameter, and anionic species information, and the reaction yield of the compound, and
wherein the parameter generation unit generates the comprehensive combination of the zeolite parameter, the metal cation parameter, and the anionic species information to be input to the trained machine learning model.

3. The design support device according to claim 1 or 2, wherein the reaction yield of the compound includes at least one of propylene yield, an olefin/paraffin ratio, and useful component yield.

4. A learning device for training a machine learning model, comprising:
a training data set acquisition unit that acquires a training data set representing correspondence relationship between a zeolite parameter and a metal cation parameter and reaction yield of a compound, a metal cation being supported by ion exchange; and
a training unit that trains the machine learning model based on the training data set by using the zeolite parameter and the metal cation parameter as explanatory variables and the reaction yield of the compound as an objective variable.

5. The learning device according to claim 4, wherein the training data set represents the correspondence relationship between the zeolite parameter, the metal cation parameter, and anion species information, and the reaction yield of the compound, and
wherein the training unit trains the machine learning model based on the training data set by using the zeolite parameter, the metal cation parameter, and the anionic species information as explanatory variables and the reaction yield of the compound as the objective variable.

6. The learning device according to claim 4 or 5, wherein the reaction yield of the compound includes at least one of propylene yield, an olefin/paraffin ratio, and useful component yield.

7. A design support method for supporting design of a metal-supported zeolite catalyst using a machine learning model in which a computer has learned correspondence relationship between a zeolite parameter and a metal cation parameter, and reaction yield of a compound, a metal cation being supported by ion exchange, comprising:
a parameter generation step of generating a comprehensive combination of the zeolite parameter and the metal cation parameter;
a prediction step of predicting the reaction yield of the compound corresponding to the combination by using a trained machine learning model; and
a candidate output step of outputting data of a candidate metal-supported zeolite catalyst based on the predicted reaction yield of the compound corresponding to the combination.

8. A design support method executed by a computer for training a machine learning model, comprising:
a training data set acquisition step of acquiring a training data set representing a correspondence relationship between a zeolite parameter and a metal cation parameter, and reaction yield of a compound, a metal cation being supported by ion exchange; and
a training step of training the machine learning model based on the training data set by using the zeolite parameter and the metal cation parameter as explanatory variables and the reaction yield of the compound as an objective variable.

9. A program causing a computer, for supporting design of a metal-supported zeolite catalyst using a machine learning model that has learned correspondence relationship between a zeolite parameter and a metal cation parameter, and reaction yield of a compound, a metal cation being supported by ion exchange, to execute:
a parameter generation procedure of generating a comprehensive combination of the zeolite parameter and the metal cation parameter;
a prediction procedure of predicting the reaction yield of the compound corresponding to the combination by using a trained machine learning model; and
a candidate output procedure of outputting data of a candidate metal-supported zeolite catalyst based on the predicted reaction yield of the compound corresponding to the combination.

10. A program causing a computer for learning a machine learning model to execute:
a training data set acquisition procedure of acquiring a training data set representing correspondence relationship between a zeolite parameter and a metal cation parameter, and reaction yield of a compound, a metal cation being supported by ion exchange; and
a training procedure of training the machine learning model based on the training data set by using the zeolite parameter and the metal cation parameter as explanatory variables and the reaction yield of the compound as an objective variable.
